# EUROPEAN PATENT APPLICATION

(11) **EP 3 536 660 A1**
(43) Date of publication of application: **11.09.2019**
(21) Application number: 18159987.9
(22) Date of filing: 05.03.2018
(51) Int. Cl.: B81C 1/00, B82Y 40/00, G01N 33/487

(54) **SELF-ALIGNED NANOPORE ON FIN**

(71) Applicant: IMEC vzw, 3001 Leuven (BE)
(72) Inventor: CHAN, Boon Teik, 3001 Leuven (BE); TAO, Zheng, 3001 Leuven (BE); DE MARNEFFE, Jean-Francois, 3001 Leuven (BE); CHEN, Chang, 3001 Leuven (BE)
(74) Representative: AWA Sweden AB

(57) **Abstract**

According to an aspect of the present inventive concept there is provided a method for forming a nanopore (10) in a semiconductor fin (20). The method comprises the steps of providing (S10) a fin structure (100) comprising at least a bottom layer (110) and a top layer (120), pattering (S20) the top layer to form a pillar (122), laterally embedding (S30) the pillar in a filler material (130), forming (S40) an aperture (140) in the filler material by removing the pillar, and forming (S50) the nanopore (10) in the bottom layer by etching through the aperture.

## Description

### Technical field

The present inventive concept relates to a method of forming a nanometre sized opening in a semiconductor structure that can be used for sensing polymers, and in particular the individual bases of deoxyribonucleic acid (DNA) or ribonucleic acid (RNA).

### Background

In the prior art, long chain polymers such as e.g. DNA may be characterised by means of a semiconductor device similar to a field-effect transistor, such as e.g. a MOSFET, comprising a drain region, a source region and an opening through which the polymer can pass. The voltage potential applied across the drain and source regions creates a gradient that causes the polymer to move through the opening. In case of e.g. a DNA or RNA strand, the sequence of bases may induce charges which form a channel between the drain and source regions, resulting in a current variation that can be detected by e.g. a current meter. Each different type of bases A, C, G and T (in case of a DNA strand) may induce a current having a particular magnitude and waveform representative of the particular charge associated with the respective type of bases. Thus, by studying the current variation as the sample passes through the opening, the sequence of bases can be detected.

This technology requires an opening of a highly controlled size and shape. Depending on the type of polymer strand to be sensed, the opening should preferably be configured to have a diameter small enough to allow only one stand to pass through the opening at any given time. DNA sequencing, for example, may require an opening having a diameter within the nanometre range, such as within the range of about 1 nm to about 10 nm. Forming such small openings are technically challenging, not at least in terms of alignment, and therefore a need exists for improved and more accurate methods for forming such semiconductor devices.

### Summary

An objective of the present inventive concept is to provide a method for forming a nanometre sized opening, or nanopore, in a semiconductor device. A further objective is to provide a method that enables a nanopore to be formed in a semiconductor fin structure, and preferably a fin having a width of 10 nm or less. Further and alternative objectives may be understood from the following.

According to a first aspect of the present inventive concept there is provided a method for forming a nanopore in a semiconductor fin, comprising:
providing a fin structure comprising at least a bottom layer and a top layer,
patterning the top layer to form a pillar,
laterally embedding the pillar in a filler material,
forming an aperture in the filler material by removing the pillar, and
forming the nanopore in the bottom layer by etching though the aperture.

The inventive method makes use of a multiple patterning process that enables a nanopore to be correctly aligned and formed on a fin having a width of the same order as the nanopore, such as about 10 nm or less. In the multiple patterning process, the position of the nanopore is aligned in a width direction, or lateral direction of the fin in a first step and aligned in a length direction of the fin in another step. The present inventive concept is based on the realisation that by employing a pillar structure formed of a top layer and a bottom layer, and cutting the top layer into a pillar structure, the pillar structure can be used as a sacrificial structure for forming an etch aperture that is self-aligned in the lateral direction of the fin. The present process allows for an improved definition and positioning of the nanopore compared to etch the nanopore in a single lithographic step. Thus, by utilising self-alignment to position the etch mask in the lateral direction of the fin, a semiconductor structure resembling a fin field-effect transistor (finFET) is enabled, which is small enough to be used for sensing polymers such as e.g. DNA or RNA strands. In such a device, an opening or pore having a width of about 1 nm to about 10 nm may be arranged between the drain region and the source region of the fin to form a channel as the sample moves through the opening. The present inventive concept is advantageous in that it allows for the lateral alignment of the nanopore to be improved, thereby allowing for a reduced ratio between the fin width and the nanopore diameter. A reduced fin width, or a reduced cross-section of the fin, is advantageous in that it may reduce the drive current required during operation of the device.

By 'pillar' is meant a structure extending in vertical direction. The pillar may also be referred to as a dot or mask feature that can be used as a sacrificial structure for forming the aperture through which the nanopore is etched.

As used herein, the term 'vertical' (for instance with reference to a direction or a plane or the pillar) denotes a geometrical axis being parallel to a stacking direction of the layers of the fin structure. Correspondingly, a 'vertical' axis is perpendicular to a main plane of extension or a main surface of any of the layer of the device, such as the bottom layer or the top layer, or a substrate on which the device may be formed. Terms such as 'above' and 'under' as used herein may accordingly refer to opposite directions along the vertical axis, with respect to a reference. As herein, the term 'horizontal' denotes a horizontal axis being perpendicular to the vertical axis.

The device resulting from the method may include a substrate supporting the afore-mentioned layers forming the fin structure. In this case, a 'vertical' direction/plane may be understood as a direction/plane being perpendicular to a main plane of extension or a main surface of the substrate. Correspondingly, a 'horizontal' direction/plane may be understood as a direction parallel to a main plane of extension or a main surface of the substrate.

The terms 'fin' or 'fin-structure' may refer to a fin-shaped feature having a length and width extension in the horizontal direction and a height extension in the vertical direction. The width direction may also be referred to as a lateral direction of the fin. The fin-shaped features may be formed using standard fin-based processes, in which for example the stacked structure of the bottom layer and the top layer may be provided with trenches defining and separating the fins.

The term 'nanopore' refer to an opening or channel extending in, and preferably through, the fin. The length extension of the opening may be oriented in the vertical direction. The prefix 'nano' refer to the fact that a diameter, or width or cross sectional size of the pore may lie within the range of about 1 nanometer to about 10 nanometer. The nanopore may have a square-shaped or circular cross section, or anything in between. Further examples will be discussed in the following in connection with some of the embodiments.

According to an embodiment, a line mask may be used to form the top layer of the fin structure into the pillar. The line mask may extend in a direction intersecting the length direction of the fin, such as for example orthogonal to the fin, such that the region in which the line mask overlaps the material of the top layer defines the etch mask used when cutting the top layer into the pillar. The resulting pillar may thus be self-aligned in the lateral direction on the fin, whereas the lengthwise position may be determined by the lithographic process forming the line mask.

According to an embodiment, the step of forming the aperture in the filler material may further include lining the aperture with a spacer material, thereby reducing a size of the aperture. The spacer material may for example be provided in an atomic layer deposition (ALD) process or by means of oxidation of the sidewalls of the aperture. Adding the spacer material allows for the size of the resulting nanopore to be reduced to a desired size, such as down to a diameter as small as 1 nm. The spacer material may for example be silicon dioxide (SiO₂), silicon nitride (Si₃N₄), silicon oxycarbide (SiOC) or a metal such as titanium nitride (TiN), tantalum nitride (TaN) or aluminum nitride (AlN).

According to an embodiment, the top layer may comprise a first mask material and a second mask material arranged on top of the first mask material, wherein the first material is an etch stop material protecting the bottom layer during etching of the second mask material. This allows for a better process control of the top layer patterning, resulting in a reduced risk of etch-back of the bottom layer during the pillar formation. Hence, a method is obtained which is compatible with a bottom layer having a reduced thickness.

According to an embodiment, the first mask material may be formed of SiO₂, Si₃N₄, SiOC, and silicon oxynitride (SiON). Depending on the type of material selected for the first mask material, the second mask material may be formed of amorphous silicon (a-Si), TiN, SiO₂, Si₃N₄, SiOC, and silicon oxynitride (SiON). Preferably, the first mask material and the second mask material are selected so as to achieve an etch selectivity between the mask layers, thereby enabling an etch stopping effect of the double mask. Such combinations may for example be a first mask material of SiO₂ combined with a second mask material of a-Si. This is particularly advantageous when the bottom layer, forming the fin, is formed of Si, since the intermediate first mask material of SiO₂ allows for the pillar pattern to be transferred into the second mask material without damaging or etching back the underlying Si fin.

The filler material may be selected such that an etch selectivity to the pillar material is achieved. The filler material may for example be SiO₂ or Si3N4.

The bottom layer, of which the resulting fin may be formed, may for example be formed of silicon, such as the top silicon layer of a silicon on insulation (SOI) substrate.

The nanopore may in one example have a substantially uniform diameter, or cross section, along its length. However, other configurations are also possible. In an embodiment, the nanopore may have a tapered or funnel-shaped profile, i.e., a diameter that decreases towards the bottom or base of the fin. Such a gradually reduced opening size may allow for an improved control of the flow through the opening, preferably such that only one sample strand at the time is guided through the opening. The tapered profile may for example be provided by means of wet etching process, such as potassium hydroxide (KOH) etching, resulting in a V-shaped profile along the (111) planes (in case of Si). Such a profile may comprise a facet of 54.7° to the silicon surface. Other examples include reactive ion etching (RIE), which may be sequenced with sidewall passivation, using for example polymerization, to achieve the desired cross sectional profile of the opening.

It will be appreciated that the forming of the nanopore may further comprise material deposition steps in order to yield even smaller openings. This may include thermal oxidation or anodic oxidation, which has the advantage of enabling the size of the opening to be monitored during the oxidation, and ALD deposition similar to the processed discussed above. Thus, according to an embodiment the step of forming the nanopore in the bottom layer may comprise the additional step of lining the nanopore with a spacer material so as to further reduce the size of the nanopore.

According to a second aspect, a semiconductor fin comprising a nanopore generated by using a method according to the first aspect is provided.

According to a third aspect, a transistor structure for DNA sensing is provided, wherein the transistor structure comprises a semiconductor fin according to the second aspect.

The devices according to the second and third aspects may generally present the same or corresponding advantages as the method according to the first aspect and the embodiments associated therewith. Thus, it is appreciated that the embodiments discussed in connection with the first aspect are equally combinable with the devices according to the second and third aspects, and are therefore not repeated in the present disclosure.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Figures 1a-e illustrate a method for forming a nanopore in a semiconductor fin.
Figures 2a-d illustrate a variation of a method for forming a nanopore in a semiconductor fin.
Figure 3 illustrate an example of a device comprising a semiconductor fin and a nanopore.
Figure 4 is a flowchart schematically illustrating an example method for forming a nanopore in a semiconductor fin.

### Detailed description

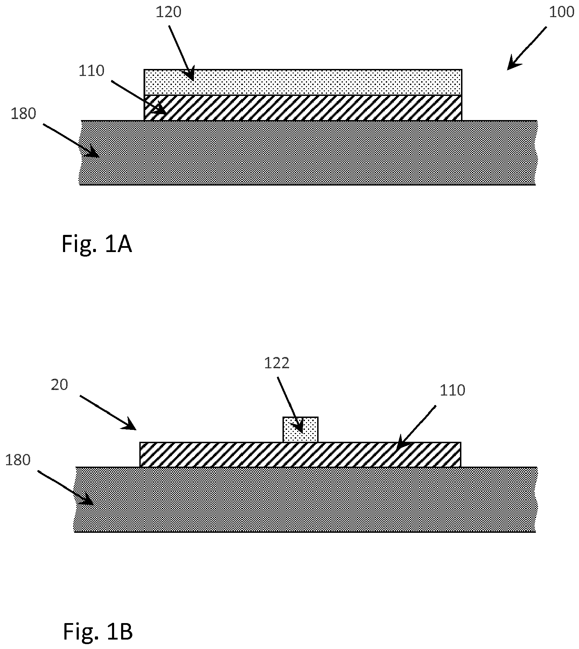

A method for forming a nanopore in a semiconductor fin will now be described with reference to figures 1a-e, illustrating vertical cross sections taken along the length direction of the fin structure.

Figure 1a shows a fin structure 100 comprising a bottom layer 110 and a top layer 120 arranged on a substrate 180. The bottom layer 110 may for example be formed of Si, such as of a SOI wafer, and the top layer 120 of for example SiO₂, Si₃N₄ or other materials which, preferably, provides an etch selectivity relative the material of the bottom layer 110. The bottom layer 110 and the top layer 120 may be provided in the form of a stack, which may be patterned into one or several fin structures 100 by means of a fin etch process readily known to a person skilled in the art.

In a subsequent step, the top layer 120 may be patterned into a pillar structure 122 as shown in figure 1b. This may for example be achieved by means of a line mask (not shown) provided across the fin structure 100 and a following etch process, leaving a pillar or dot shaped mask structure 122 on top of the fin 20 formed by the bottom layer 110. The position of the line mask in the length direction of the fin structure 100 determines the alignment of the pillar structure 122 along the fin 20, whereas the pillar structure 122 is self-aligned in the lateral direction (i.e., the width direction) of the fin 20.

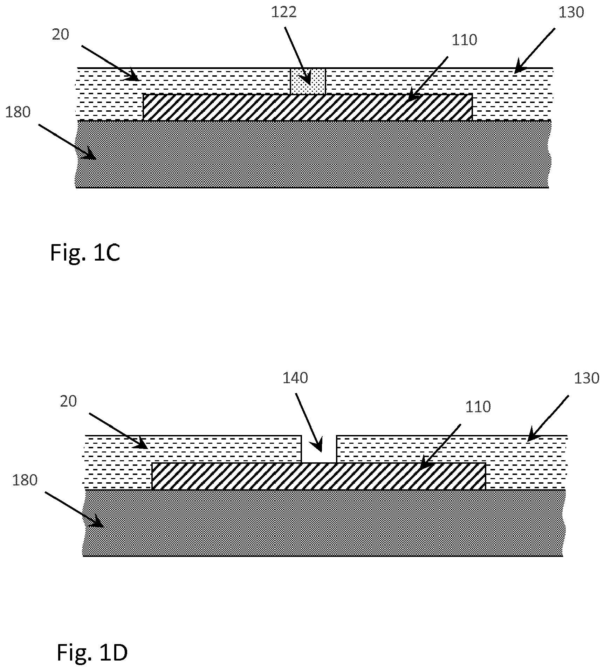

In figure 1c, the pillar 122 has been embedded in a filler layer 130, which for example may be formed of a dielectric filling material such as Si₃N₄ of SiO₃. In the present example, the top surface of the structure has been planarized, for example by means of a chemical-mechanical planarization (CMP) process.

Figure 1d shows the structure after the pillar 122 has been removed, leaving an aperture 140 that can be used as an etch mask for the resulting nanopore 10. The pillar 122 may hence be referred to as a sacrificial pillar 122. The material of the pillar 122 may for example be etched in a wet etch process, such as TMAH in case the pillar 122 is formed of for example amorphous Si. Advantageously, the TMAH etch is used in combination with an etch stop layer separating the pillar 122 and the underlying fin material.

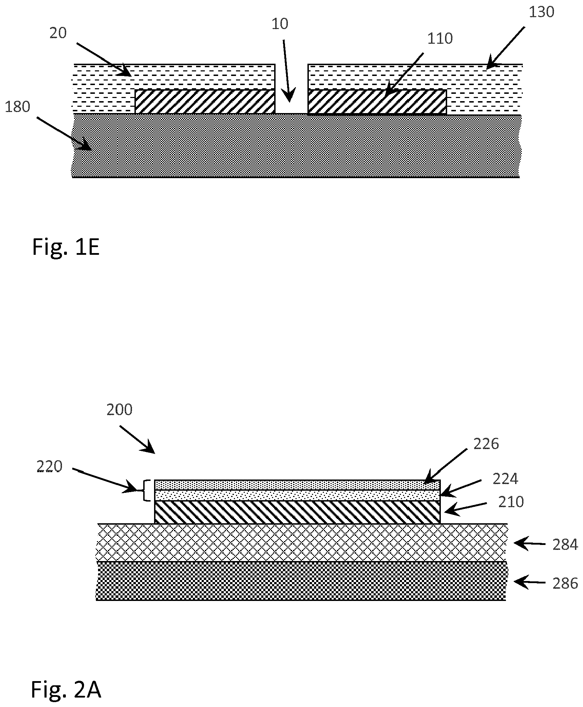

The pattern defined by the aperture 140 in the filler material layer 130 may then be transferred into the fin 20 to form the nanopore 10. An example of the resulting device is illustrated in figure 1e, depicting the semiconductor fin 20 when provided with the nanopore 10 extending in a top-bottom direction of the fin 20. The nanopore 10 is self-aligned in the width direction of the fin 20. The self-alignment is achieved due to the use of the sacrificial pillar 122 which is formed of the top layer 120 of the fin structure 100.

The above-described example method for forming the nanopore may be varied in several ways, using different material combinations, material layers and processing steps. An example of such variation will now be discussed with reference to figures 2a-e. It will be appreciated that the depicted examples may be similarly configured as the embodiments described above in connection with figures 1a-e.

Figure 2a shows a cross sectional side view of a fin structure 200 comprising a bottom layer 210 of for example Si, arranged on a substrate comprising a dielectric layer 284, such as for example SiO₂, and a Si layer 282. The bottom layer 210, the dielectric layer 284 and the Si layer 282 may in some examples be formed of an SOI wafer.

The fin structure 200 may further comprise a first mask material 224 and a second mask material 226, which together may form the top layer 220 of the fin structure 200. The first mask material 224 may be arranged between the bottom layer 210 and the second mask material 226 so as to form an interface between the top layer 220 and the bottom layer 210. In this way, the top layer 210 may be referred to as a dual mask. The first mask material 224 may in some examples be a dielectric such as SiO₂, Si₃N₄, SiOC or SiON. The second mask material 226 may be amorphous Si, TiN, Si₃N₄, SiO₂, SiOC or SiON, depending on the type of material used in the first mask 224. Preferably, the first and second mask materials 224, 226 are selected such that the combination allows for an etch selectivity between the materials, which advantageously allows for the first mask material 224 to act as an etch stop layer during the formation of the pillar 222. In the present example, the first mask material is SiO₂ and the second mask material amorphous Si.

The forming of the pillar 222 requires the top layer, and in this example the second mask material 226, to be patterned into the desired structure. The patterning may for example be performed by means of a lithography and etching. For this purpose, a hard mask of for example spin-on-carbon (SoC) and spin-on-glass (SoG) may be used (not shown). Alternatively, DARC (SiOC) may be provided on an APF (carbon film) stack. Photoresist may be employed to pattern the SoC and SoG layers into a line mask 250 that can be used when cutting the second mask material 226 of the fin structure 200 into a self-aligned pillar or dot structure, using the fist mask material 224 as an etch stop.

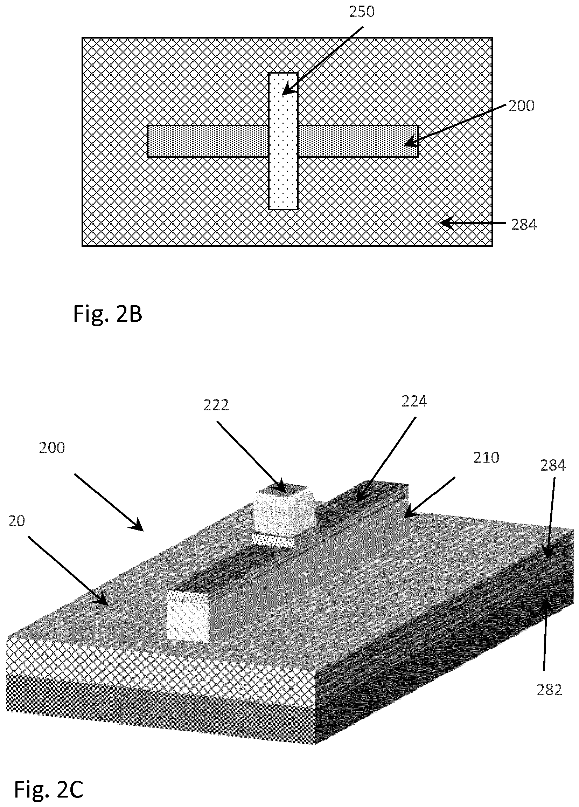

Figure 2b shows a top view of the fin structure 200 of figure 2a, in which the line mask 250 crosses the fin structure 200 in a substantially orthogonal manner.

A perspective view of the fin structure 200 is shown in figure 2c. The fin structure 200 comprises a fin 20 formed of the bottom layer 210, a pillar 222 arranged on top of the fin 20, and the etch stop layer 224 arranged in between. The figure indicates that the etch stop layer 224 has been slightly etched back during the forming of the pillar 222. It should be noted that the pillar 222 may as well be formed without the intermediate etch stop layer 224, i.e., formed from a stack comprising only the bottom layer and the top layer. In that case, it might be advantageous to use a slightly thicker bottom layer so as to compensate for possible etch back effects.

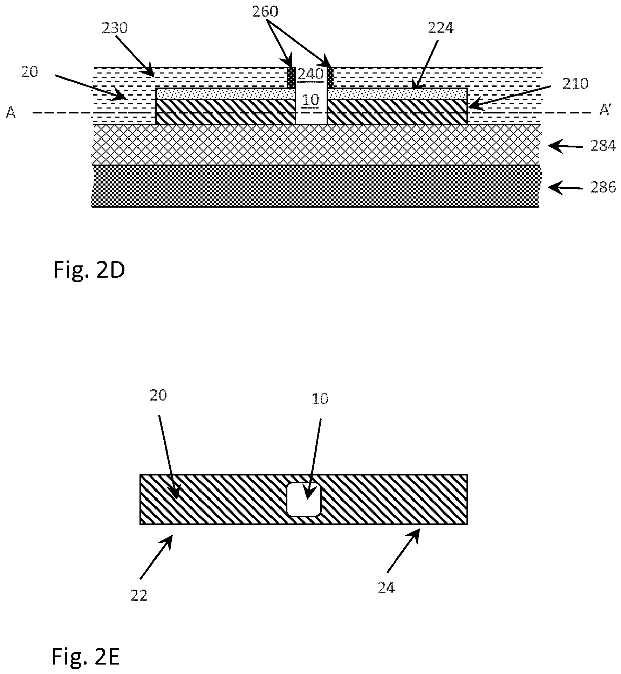

Figure 2d is a cross sectional side view of the fin shown in figure 2c, after the pillar 222 has been embedded in the filler material 230 and replaced by the aperture 240. As also shown in the present figure, the aperture 240 may be lined with a spacer material 260 provided by for example oxidation or ALD. The spacer material 260 may be added so as to reduce the diameter of the aperture 260 and thereby enable a smaller nanopore 10 to be formed in the fin 20. Even though not shown in the present cross section, the spacer material 260 may be provided on all sidewalls of the aperture 240, which hence may have a width or diameter that is smaller than the width of the fin 20. This allows for the nanopore 10 to be etched through the fin material without breaking through the sidewalls of the fin 20. The spacer material 260 may for example be a dielectric such as SiO₂, Si₃N₄, SiOC, TiO₂, ZrO₂ or HfO₂ or a metal such as TiN, TaN or AIN.

Figure 2e is a cross sectional top view of the fin 20 shown in figure 2d, taken along the line A-A'. As shown in the present figure, the nanopore may be centrally located in the width direction of the fin 20, allowing a polymer such as e.g. a DNA or RNA strand to pass through the fin and thereby form a channel between the drain region 22 and the source region 24 of the fin 20. In the present example, the fin 20 may have a width of about 10 nm and a height of about 10 nm to about 20 nm. The nanopore 10 may in the present example have a width of about 5 nm.

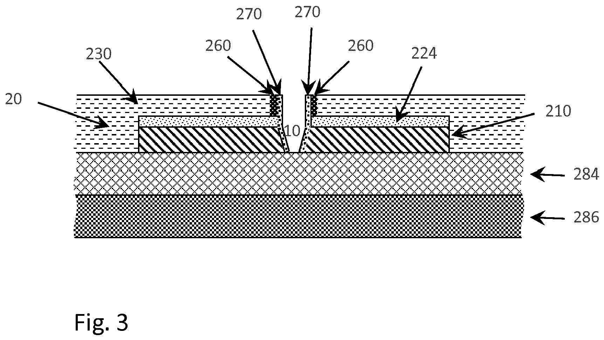

Figure 3 illustrates a semiconductor fin 20 according to another example. The semiconductor fin 20 may be similarly configured as the previously described embodiments, but differ in that the nanopore 10 is provided with a tapered or funnel shaped profile. As shown in the present figure, the opening 10 may decrease towards the base of the fin 20. This tapered profile may for example be achieved by a RIE process, in which etching is cycled with sidewall passivation. Alternatively, or additionally an anisotropic wet etch, such as e.g. orientation dependent KOH etch of Si, may be employed to provide a V-shaped profile of the opening 10.

Further, the nanopore 10 may be provided with a spacer material 270 lining the sidewalls of the nanopore 10. The spacer material 270 may be similar to the one used for the aperture 140, and allows for the diameter of the pore 10 to be even further decreased.

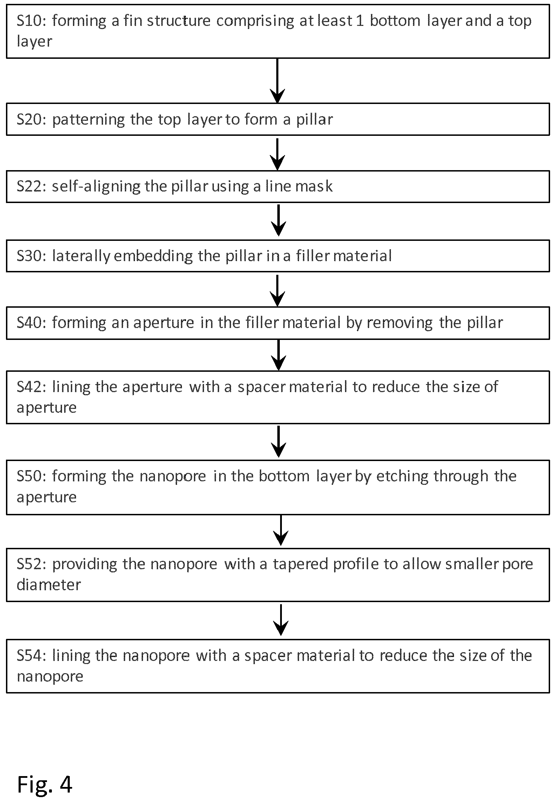

Figure 4 is a schematic outline of a method according to embodiments described above. The method may comprise the steps of providing S10 a fin structure comprising at least a bottom layer and a top layer, patterning S20 the top layer to form a pillar, laterally embedding S30 the pillar in a filler material, forming S40 an aperture in the filler material by removing the pillar, and forming S50 the nanopore in the bottom layer by etching through the aperture. The patterning S20 the top layer to form a pillar may include a self-aligning S22 of the pillar using a line mask. Further, the aperture may be lied S42 with a spacer material to reduce the size of the aperture and hence the nanopore. This also applies to the nanopore itself, which may be lined S54 with the same or a similar material. In some embodiments, the nanopore additionally, or alternatively, may be provided with a tapered S52 profile so as to allow for an even smaller pore diameter to be achieved.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. Method for forming a nanopore (10) in a semiconductor fin (20), comprising:
a) providing (S10) a fin structure (100) comprising at least a bottom layer (110) and a top layer (120);
b) pattering (S20) the top layer to form a pillar (122);
c) laterally embedding (S30) the pillar in a filler material (130);
d) forming (S40) an aperture (140) in the filler material by removing the pillar, and
e) forming (S50) the nanopore (10) in the bottom layer by etching through the aperture.

2. The method of claim 1, wherein the step b) comprises self-aligning (S22) the pillar on the bottom layer using a line mask (150) intersecting the fin structure.

3. The method of claim 1 or 2, wherein step d) further comprises lining (S42) the aperture with a spacer material (160), thereby reducing a size of the aperture.

4. The method of any one of the preceding claims, wherein the top layer comprises a first mask material (124) and a second mask material (126) arranged on top of the first mask material, wherein the first mask material is an etch stop material protecting the bottom layer during etching of the second mask material.

5. The method of claim 4, wherein the first mask material is selected from the group consisting of: silicon dioxide, SiO₂; silicon nitride, Si₃N₄; silicon oxycarbide, SiOC; and silicon oxynitride, SiON, and wherein the second mask material is selected from the group consisting of: amorphous silicon, a-Si; titanium nitride, TiN; silicon dioxide, SiO₂; silicon nitride, Si₃N₄; silicon oxycarbide, SiOC; and silicon oxynitride, SiON.

6. The method of any one of the preceding claims, wherein the filler material is selected from the group consisting of: silicon dioxide, SiO₂; and silicon nitride, Si₃N₄.

7. The method of any one of the preceding claims, wherein the spacer material is selected from the group consisting of: silicon dioxide, SiO₂; silicon nitride, Si₃N₄; silicon oxycarbide, SiOC; and a metal.

8. The method of any one of the preceding claims, wherein the step e) comprises forming (S52) a tapered nanopore.

9. The method of any one of the preceding claims, wherein the bottom layer comprises silicon.

10. The method of any one of the preceding claims, wherein the step e) comprises lining (S54) the nanopore with a spacer material (170), thereby reducing a size of the nanopore.

11. A semiconductor fin (20) comprising a nanopore (10) formed by a method according to any one of the preceding claims.

12. A transistor structure for DNA sensing, comprising a semiconductor fin according to claim 11.
